(19) Europäisches Patentamt
European Patent Office
Office européen des brevets

(11) Veröffentlichungsnummer: **0 323 494 B1**

(12) # EUROPÄISCHE PATENTSCHRIFT

(45) Veröffentlichungstag der Patentschrift: **19.01.94**

(51) Int. Cl.[5]: **B01F 17/00**, A61K 9/10

(21) Anmeldenummer: **88905345.0**

(22) Anmeldetag: **27.06.88**

(86) Internationale Anmeldenummer:
**PCT/CH88/00114**

(87) Internationale Veröffentlichungsnummer:
**WO 89/00077 (12.01.89 89/02)**

Die Akte enthält technische Angaben, die nach dem Eingang der Anmeldung eingereicht wurden und die nicht in dieser Patentschrift enthalten sind.

(54) **LECITHINGELE.**

(30) Priorität: **01.07.87 CH 2472/87**

(43) Veröffentlichungstag der Anmeldung:
**12.07.89 Patentblatt 89/28**

(45) Bekanntmachung des Hinweises auf die Patenterteilung:
**19.01.94 Patentblatt 94/03**

(84) Benannte Vertragsstaaten:
**AT BE CH DE FR GB IT LI LU NL SE**

(56) Entgegenhaltungen:
**EP-A- 0 227 012**
**WO-A-86/02264**
**DE-C- 388 023**

**Goldschmidt Informiert, Band 57, 1982, (Essen, DE), M.Stupar et al.:"Herstellung von Mikroemulsionsgelen mit TEGOR - Tensiden", Seiten 22-28**

**Voigt: Lehrbuch der pharmazeutischen Technologie 1973, (Berlin, DE) Seite 382, Abschnitt 23.5.5.2 Lezithine**

(73) Patentinhaber: **ZAMBON GROUP S.p.A.**
**Via Lillo del Duca 10**
**I-20091 Bresso-Milano(IT)**

(72) Erfinder: **LUISI, Pier Luigi, Prof. Dr.**
**Institut für Polymere ETH-Zentrum**
**CH-8092 Zürich(CH)**

(74) Vertreter: **Zink-Wild, Markus Peter**
**Patentanwaltsbüro Zink,**
**Birchlistrasse 11**
**CH-8173 Riedt-Neerach (Zürich) (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 323 494 B1

## Beschreibung

### Stand der Technik

Gele sind Materialien, die auf vielen Gebieten der Technologie grosse Verwendung finden, z.B. in der Lebensmittelindustrie (Gelatingele), in der Kosmetik und Pharmakologie (die verschiedenen Salben, die als Gele verwendet werden), in der Photographie, bei den Trennungsverfahren (Chromatographie-Gele) und so weiter.

Der Ausdruck Gel wird heutzutage in der Technik wie auch in der wissenschaftlichen Literatur in einem ziemlich breiten und unspezifischen Sinn benutzt. In striktem Sinne sollte man dann von Gelen sprechen, wenn ein makromolekulares System vorliegt, in welchem die Ketten miteinander vernetzt sind, und welches eine unendliche Viskosität besitzt ( " a phase that is largely liquid but incapable of flow because it is held rigid by molecular chains, usually cross-linked, that pass through it", wie definiert in "International dictionary of medicine and biology", Vol. II). Unter "Gele" versteht man jedoch viel häufiger einfach eine hochviskose Masse, und es werden dann die verschiedensten Definitionen angegeben. Wenn man z.B. bei der englischen Literatur bleibt, findet man folgendes:

" a two-phase colloidal system consisting of a solid and a liquid" ( McGraw-Hill Encyclopedia of Chemistry, S.P.Parker edit., McGraw Hill Book Company, New York);
oder " a colloidal solution of a liquid in a solid" ( Grant and Hackh's Chemical Dictionary, fifth edition, Mc Graw-Hill Book Company, New York);
oder ," a colloid in which the disperse phase has combined with the continous phase to produce a viscous Jelly-like product" (Hawley's Condensed Chemical Dictionary, 11th Edition, Van Nostrand Reihnold Company, New York);
oder " a colloidal system with a finite, usually rather small, yield stress" (Pure and Appl. Chem., 31, 1972, S. 606)

Und wenn man auf die deutsche Literatur angewiesen ist, findet man auch eine Vielfalt von verschiedensten Definitionen, z.B.
" von Gelatine abgeleitete Bezeichnung aus der Kolloidchemie für formbeständige, leicht deformierbare, an Flüssigkeiten und Gasen reiche disperse Systeme aus mindestens zwei Komponenten, die zumeist aus einem festen, kolloid zerteilten Stoff mit langen oder stark verzweigten Teilchen und einer Flüssigkeit (meist Wasser) als Dispersionsmittel bestehen" (Römpps Chemie-Lexikon, 8. Auflage, Franckh'sche Verlagshandlung, Stuttgart);
oder "ein disperses System, bei dem die dispersen Bestandteile netz-oder wabenartig im Dispersionsmittel angeordnet und z.T. an den Berührungstellen miteinander verbunden sind" (Fachlexikon ABC Chemie, Band 1 A-K,Verlag Harri Deutsch, Frankfurt, 1976).

Man unterscheidet zwischen chemischen und physikalischen Gelen (letzere sind solche, bei welchen die Gelstruktur nur durch physikalische Kräfte aufrecht erhalten wird, im Gegensatz zur chemisch kovalenten Vernetzung).

Es ist dann häufig so, dass Gele einfach als hochviskose Masse definiert werden, die eine hohe aber messbare (im Gegensatz zur unendlichen) Viskosität aufweisen. Diese hochviskose Masse wird dann im Detail bezüglich rheologischer Eigenschaften untersucht (siehe zum Beispiel A.H. Clark und S.B. Ross-Murphy, in Adv. Polymer Sci., 83, 1987, S.61 ).

In der Regel werden die Gele aus einer Lösung erhalten, wobei ein Verfahren angewendet wird, welches diese Lösung zur Erstarrung (Gelierung) bringt. Die Viskosität der hochviskosen Masse ist in der Regel um einige Zehnerpotenzen höher als die Viskosität der ursprünglichen Lösung. Die Bezeichnung "Gel" wird oft verwendet, auch wenn die Struktur der Masse nicht bekannt ist.

In dieser Schrift wird der Ausdruck "Gele" in diesem weitesten Sinne benutzt, nämlich im Sinne einer hochviskosen Masse, deren Struktur noch nicht geklärt ist.

Die am meisten bekannten und verbreiteten Gele sind die wässrigen Gele, in welchen Wasser als kontinuierliche Hauptkomponente verwendet wird, zu welcher eine Verbindung zugefügt wird, welche die Gelierung verursacht. In der Regel ist letzere ein wasserlösliches Polymer, wie z.B. Gelatine, oder ein Polysaccharid (Agarose). Es sind auch Organogele bekannt, in welchen die flüssige Hauptkomponente ein organisches Lösungsmittel ist und die Substanz, welche die Gelierung verursacht, ein apolares Polymer ist.

Vor kurzem ist eine spezielle Familie von Organogelen beschrieben worden, die sogenannten Mikroemulsionsgele, in welchen die flüssige organische Hauptkomponente eine Wasser-in-Oel Mikroemulsion ist, d.h. eine Lösung eines Tensides in einem organischen Lösungsmittel.

In diesem Falle geht man typischerweise von dem System AOT-Isooktan-Wasser aus (AOT ist das anionische Tensid bis(2-ethyl-hexyl) Natriumsulfosuccinat) wobei Gelatine in der wässrigen Mikrophase

gelöst ist, und die Erstarrung des ganzen Systems induziert. Man braucht relativ viel Wasser, typischerweise 10-20 vol% bezüglich organisches Lösungsmittel. Solche Gele wurden in einem Patent (WO 86/02264) und später in einigen Veröffentlichungen beschrieben (G. Haering und P.L. Luisi, J.Phys. Chem.,90, 1986, S. 5892; C. Quellet und H.F.Eicke, Chimia, 40, 1986, S. 7).

Solche AOT-Isooktan Mikroemulsionsgele können aber als solche nicht in der Pharmakologie oder in der Nahrungsmittelindustrie Verwendung finden, weil sie nicht biokompatibel sind (wegen der grossen Menge an Alkanen und wegen AOT, beide toxisch).

Um ein biokompatibles Organogel herstellen zu können, müssen sowohl das organische Lösungsmittel, wie auch der gelierende Wirkstoff biokompatibel sein. Solche Gele waren bis jetzt noch nicht bekannt.

Man findet in einer deutschen Publikation die Information, dass Gele aus Lecithin in Benzol (nur in diesem Lösungsmittel) gebildet werden können, und nur indem man die Lösung unterkühlt (H. Frischleider, G. Kleser, R. Lochman, R. Misselwitz und D. Zirwer, Chem. and Phys. Lipids, 21, 1978, S. 131).

Wir haben jetzt mit Ueberraschung festgestellt, dass die Zugabe von minimalen Mengen Wasser zu einer Lösung von gut gereinigtem Lecithin in organischen Lösungsmitteln, wobei die Molarität des zugegebenen Wassers in einem genauen stöchiometrischen Verhältnis zu der Molarität des Lecithins steht, die Fähigkeit aufweist, die ganze organische Lösung in eine hochviskose Masse erstarren lässt.

Die wichtigste chemische Verbindung in der Erfindung ist Lecithin. Lecithin (oder Lezithin), ist der triviale Name für Phosphatidylcholin, ein Phospholipid, welches zur Gruppe der Glycerinphosphatide gehört. Wie im Lehrbuch für Biochemie von P. Karlson angegeben (Kurzes Lehrbuch der Biochemie, Georg Thieme Verlag, Stuttgart, S. 211), ist der Grundbaustein aller Glycerinphosphatide die sn-Glycerin-3-Phosphorsäure. Werden die beide freien Hydroxyle nun mit zwei langkettigen Fettsäuren verestert, so entsteht eine Phosphatidsäure. Im Phosphatidylcholin (oder Lecithin) ist die zweite saure Gruppe der Phosphorsäure noch mit einem Aminoalkohol verestert, dem Cholin.

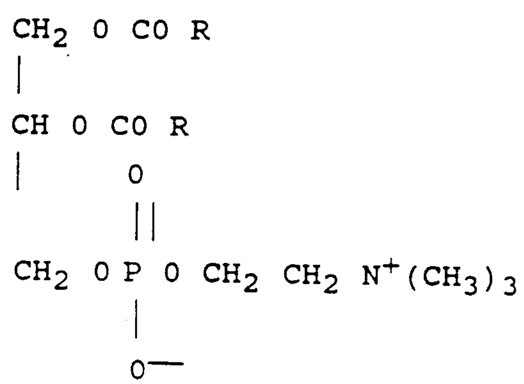

Die Definition von Lecithin ist unabhängig von der Natur von R, obwohl natürlich vorkommende Lecithine bevorzugt Carboxylsäuren mit einer geraden Anzahl von C-Atomen zwischen 16 und 22 enthalten. Die Gruppe R kann auch eine oder mehrere Doppelbindungen enthalten. Natürliche Lecithine, z.B. aus dem Eigelb oder der Sojabohne enthalten in der Regel eine Mischung von verschiedenen R Gruppen. Falls die Phosphatidylcholine synthetisch hergestellt sind, kann man von synthetischen Lecithinen sprechen. Es ist ferner zu bemerken, dass die Pharmazeuten oft den Ausdruck Lecithin als Synonym für "Lipide" oder Fette benutzen.

Die Erfindung, die hier beschrieben wird, ist durch die Merkmale in den unabhängigen Patentansprüchen gekennzeichnet. Bevorzugte Ausführungsformen sind in den abhängigen Patentansprüchen definiert.

## Beschreibung der Methode

### Die Reinigung von Lecithin

Sojalecithin von Sigma (P 3644 Type IV) oder Eilecithin von Fluka (61755) wurden mittels Silicagel-Säulenchromatographie gereinigt. Als Laufmittel benutzten wir $CH_2Cl_2$: MeOH 1:1 (v/v). Mit dieser Methode ist es möglich, die meisten Verunreinigungen zu eliminieren. Dies ist in Fig. 1 gezeigt, welche eine Dünnschicht-Chromatographie von Lecithin darstellt: ungereinigtes Soja-Lecithin der Firma Sigma (1); dasselbe gereinigt mit unserer Methode (2); Eilecithin von der Firma Fluka ungereinigt (5) und gereinigt (4).

Das Laufmittel war Chloroform: Methanol:Wasser =65:25:4. Das erhaltene Lecithin mit einem Rf-Wert von 0.22 (Dünnschichtchromatographie in $CHCl_3$:MeOH:$H_2O$ 65:25:4 v/v/v) ist geeignet zur Herstellung der Gele.

**Das Verfahren**

Lösungen von Lecithinen in einer sehr grossen Vielfalt an Lösungsmitteln können bei Raumtemperatur in Gele umgewandelt werden, in dem man eine kritische und ganz kleine Menge Wasser zufügt (typischerweise 0.5 -1 % in v:v ).

Das Wasser im System wird hier mit der Grosse $w_o$ definiert, welche den Quotient zwischen der Molarität Wasser und der Molarität Lecithin darstellt, i.e.

**$w_o$ =[$H_2$0]/[Lecithin]**

Für die Herstellung eines Gels benötigt man eine Lecithinlösung, (z.B. 50 bis 200 mM), welche unter Rühren bei Raumtemperatur im organischen Lösungsmittel hergestellt wird. Die Zeit, um Lecithin zu lösen beträgt wenige Minuten bis einige Stunden und ist vom organischen Lösungsmittel abhängig. Einige dieser Lösungen sind klar, andere trüb, wobei letztere nach Zugabe einer minimalen Menge Wasser, z.B. $w_o$=1 klar werden. Zu diesen Lösungen wird schrittweise bei Raumtemperatur Wasser zugegeben bis $w_o$ (Gel) erreicht ist. Wir definieren mit $w_o$ (Gel) exakt jenes $w_o$, welches benötigt wird, um ein festes Gel zu erhalten.

$w_o$ (Gel) ist abhängig vom organischen Lösungsmittel und ist in der Tabelle 1 aufgeführt. Die Bildung des Gels, nach Zugabe der kritischen Menge Wasser benötigt einige Sekunden bis Minuten. Ungenügend gereinigtes Lecithin bildet keine Gele.

Merkmal dieser Gele ist eben dies, dass sie sich ganz plötzlich bilden, wenn die kritische Grenze von Wasser erreicht wird, d.h. bei $w_o$ = $w_o$ ( Gel).

Es handelt sich hier um die Erfindung von neuen Materialien, die sich in ganz vielen organischen Systemen bilden, und obwohl die Struktur noch unbekannt ist, bilden sich diese Strukturen wahrscheinlich dank einer ausgeprägten Wechselwirkung zwischen Lecithinmolekülen und Wasser.

Tabelle 1 gibt eine Übersicht über die verschiedenen ausgewählten Systeme, welche nach dem beschriebenen Verfahren geliert werden können.

Die Gelbildung erfolgt sowohl mit destilliertem Wasser, als auch mit einer Salzlösung (z.B. KCl bis zu 7.4 g/l).

Die Gelbildung ist ein reversibler Prozess. Verflüssigt man das Gel durch Erwärmen, bildet es sich nach Abkühlen auf Raumtemperatur mit seinen ursprünglichen Eigenschaften (z.B. Viskosität) zurück.

Für die physikalische Charakterisierung dieser organischen Gele wurden Scherviskositätsmessungen und NMR-Studien durchgeführt. Die Werte von ETA*, G′ und G″ bei konstanter Frequenz sind in Tabelle 2 aufgeführt.

Die chemische Verschiebung der Wasserprotonen (in ppm) ist bei drei verschiedenen $w_o$-Werten, bei wo (Gel), ferner vor und nach der Gelierung, d.h. bei $w_o$ (Gel)-1 und bei $w_o$ (Gel)+1 ebenfalls in Tabelle 2 angegeben.

Es ist möglich, Gastmoleküle in dieses Lecithin-System einzubringen.

Generell können diese, je nach Löslichkeit des Gastmoleküls, sowohl in der organischen wie auch in der wässrigen Phase lokalisiert werden. Um ein homogenes System zu erhalten, werden diese Moleküle vor der Gelierung gelöst.

Tabelle 1

Gelsysteme mit Sojalecithin [200mM]

| Lösungsmittel | $w_{o(Gel)}$ |
|---|---|
| Laurinsäureethylester | 4 |
| Laurinsäurebutylester | 7 |
| Myristinsäureethylester | 5 |
| Myristinsäureisopropylester | 3 |
| Palmitinsäureisopropylester | 3 |
| Stearinsäurebutylester | 3 |
| Isooctan | 3 |
| Cyclopentan | 8 |
| Cyclohexan | 6 |
| Cycloheptan | 7 |
| Cyclooctan | 7 |
| Cyclodecan | 12 |
| Methylcyclohexan | 7 |
| tert.-Butylcyclohexan | 4 |
| Bicyclohexyl | 4 |
| Phenylcyclohexan | 12 |

| | |
|---|---|
| 1.3.5. - Triisopropylbenzol | 3 |
| Octylbenzol | 6 |
| trans - Dekalin | 5 |
| n - Pentan | 3 |
| n - Hexan | 3 |
| n - Heptan | 2 |
| n - Octan | 2 |
| n - Nonan | 2 |
| n - Decan | 2 |
| n - Undecan | 2 |
| n - Dodecan | 1 |
| n - Tridecan | 1 |
| n - Tetratradecan | 2 |
| n - Pentadecan | 1 |
| n - Hexadecan | 1 |
| n - Heptadecan | 1 |
| 2.3-Dimethylbutan | 4 |
| 1 - Hexen | 6 |
| 1 - Octen | 4 |
| 1.7 - Octadien | 7 |

| | |
|---|---|
| (1R)-(+)-trans-Pinan | 6 |
| (1R)-(+)-cis-Pinan | 10 |
| Tripropylamin | 4 |
| Tributylamin | 2 |
| Triisobutylamin | 3 |
| Trioctylamin | 2 |
| N,N - Dioctylamin | 2 |
| Dibutylether | 6 |
| 2-Decenylbernstein-säureanhydrid | 7 |

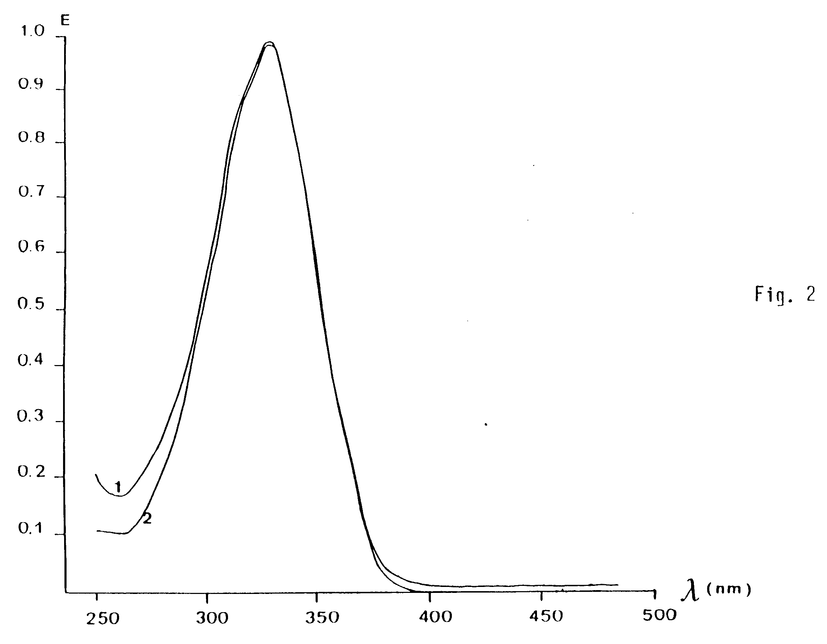

Fig. 2 zeigt das Absorptionsspektrum von Vitamin A-Palmitat, welches in der organischen Phase des Systems, in diesem Fall n-Hexadekan, gelöst ist. In der Abszisse wird die Wellenlänge, in der Ordinate die optische Dichte (direkt proportional zur Konzentration) aufgetragen. Das Spektrum des Vitamins im Gel (1) wird dem Spektrum des Vitamins in Hexadekan Lösung (2) verglichen.

Ferner integrierten wir noch anderen Biomoleküle in diesen Systemen (z.B. Vitamin C und den Farbstoff Erythrosin).

Tabelle 2

| Lösungs-mittel | $w_o$ | NMR-Daten | Scherviskositäts Daten | | |
|---|---|---|---|---|---|
| | (f) | (g) | ETA*(a,b,c) | G'(d) | G"(e) |
| Laurinsäure-ethylester | 4 | 4.5745<br>4.5657<br>4.5071 | c:8.822*10 | 7.966 | 4.338*10 |
| Laurinsäure-butylester | 7 | 4.7705<br>4.7603<br>4.7589* | c:6.294 | $1.608*10^2$ | $2.705*10^2$ |
| Myristin-säure-ethylester | 5 | 4.5571<br>4.5514<br>4.5084 | c:$1.446*10^2$ | 1.525*10 | 7.065*10 |
| Myristin-säureiso-propylester | 3 | 4.6029<br>4.5704<br>4.5650 | c:$4.146*10^2$ | 2.178*10 | $2.062*10^2$ |
| Palmitin-säureiso-propylester | 3 | 4.6248<br>4.6017<br>4.5914* | c:$1.714*10^2$ | 2.728*10 | 8.123*10 |
| Isooctan | 2 | 4.8414<br>4.8514<br>4.8601 | b:$3.702*10^4$<br>Daten für $w_o$=1:<br>c:$2.460*10^3$ | $7.700*10^3$<br><br>$6.844*10^2$ | $7.005*10^3$<br><br>$1.022*10^3$ |

Erklärung der in dieser Tabelle gebrauchten Abkürzungen:

- a : ETA* bei der Frequenz $1.077*10^{-1}$ rad/sec gemessen.
- b : ETA* " " " $2.812*10^{-1}$ rad/sec " .
- c : ETA* " " " $5*10^{-1}$ rad/sec " .

- d : G', elastische Komponente von G*, die im Zusammenhang mit der pro Schwingung wiedergewinnbaren Energieanteil steht (Speichermodul). Einheit : $dyne*cm^2$
- e : G", viskose Komponente von G*, die im Zusammenhang mit dem pro Schwingung in Wärme umgewandelten Energieanteil steht (Verlustmodul). Einheit : $dyne*cm^2$

- f : alle Daten in dieser Kolonne zeigen den Gehalt von Wasser an, nämlich $w_o$, welcher für die Gelierung notwendig ist.

- g : Normalerweise wurden die NMR-Werte bei folgendem Wassergehalt ermittelt : $w_o$(gel)-1, $w_o$(gel), $w_o$(gel)+1.
  *: Wert wurde bei $w_o$(gel)+2 genommen.
  Einheit : ppm

Beispiele

(1) **Vitamin C**

Zuerst wird eine wässrige Lösung von Vitamin C mit der Konzentration 0.3201 M hergestellt. Davon werden $w_o$=2 (≡21.66µl) entnommen und in das System Soja-Lezithin (200mM)/n-Oktan (V=3ml) zugegeben. Es wird ein klares Organogel erhalten.

(2) **Vitamin A-Palmitat**

Zu 3ml n-Hexadekan wird 16.5mg Vitamin A-Palmitat zugegeben (≡$1.05*10^{-2}$ M). Um ein sinnvolles UV-Spektrum zu erhalten, entnimmt man dieser Stammlösung 60µl und gibt diese Menge zu 2.940ml n-Hexadekan, in welchem schon vorher 0.228g Soja-Lecithin gelöst worden sind. Nachdem gut gerührt ist, wird Wasser dem System zugeführt, und zwar nur $w_o = 1$. (Da ja die Konzentration von Soja-Lecithin 100mM ist, bedeutet $w_o = 1$ in diesem Falle natürlich eine Wassermenge von 5.41µl). Das vormals trübe System wird binnen 5 Sekunden klar und die Gelierung setzt sofort ein.

Mittels UV-Spektroskopie kann bei der Wellenlänge $\lambda = 327.5$nm ein Maximum von 0.982 beobachtet werden.

Wenn man ein UV-Spektrum mit der entsprechenden Menge Vitamin A-Palmitat in n-Hexadekan aufnimmt, so erhält man ebenfalls ein Maximum bei $\lambda = 327.5$nm!

(3) **Vitamin $B_{12}$**

Als System in welchem man Vitamin $B_{12}$ inkorporieren will, wird Soja-Lecithin (200mM)/n-Oktan(V = 3ml) verwendet; um das entsprechende Gel zu erhalten, wird $w_o = 2$ an Wasser benötigt. Vitamin $B_{12}$ wird voher in Wasser gelöst und dann zugegeben. Es ist ausdrücklich zu bemerken, dass Wasser und Vitamin $B_{12}$nicht separat, sondern zusammen zugegeben werden. Es werden Organogele erhalten, welche drei verschiedene Konzentrationen an Vitamin $B_{12}$ enthalten:

- $1.624*10^{-5}$ M
- $3.209*10^{-5}$ M
- $6.099*10^{-5}$ M

Das Gel ist stabil, allerdings werden nach einem Tag "rote Punkte" sichtbar.

(4) **Cytochrome C**

Ebenfalls für Cytochrom C verwendet man als System Soja-Lecithin(200mM)/n-Oktan (V = 3ml). Um das entsprechende Gel zu erhalten, wird $w_o = 2$ an Wasser benötigt.

Cytochrom C wird wie Vitamin $B_{12}$ vorher in Wasser gelöst. Wiederum wird Cytochrom C also zusammen mit dem Wasser zugegeben.

Es kann ein Gel erhalten werden, welches 2 verschiedene Konzentrationen an Cytochrom C enthält.

- $1.50*10^{-5}$ M
- $2.50*10^{-5}$ M

Auch im Falle von Cytochrome C wird ein stabiles Gel erhalten. Allerdings zeigen sich nach einem Tag wie im Falle von Vitamin $B_{12}$ "rote Punkte".

(5) **Nifedipin**

Nifedipin ist sowohl in Wasser wie auch in organischen Lösungsmitteln schlecht oder gar nicht löslich. Für die pharmazeutische Industrie ist es von grossem Interesse, dieses Herzmittel in eine Verabreichungsformulierung zu bringen, die aus Produkten besteht, welche vom Körper abgebaut werden können.

Deshalb werden Fettsäureester verwendet, um Gele mit inkorporiertem Nifedipin zu erhalten. Es ist klar zu bemerken, dass Nifedipin in den Fettsäureestern allein schlecht oder in pharmazeutisch unbedeutenden Mengen löslich ist.

Wenn man aber zu einer entsprechenden Menge Nifedipin und 0.456g Soja-Lecithin 3ml Fettsäurester gibt und gut rührt, so erhält man nach 30 Minuten eine klare, gelbe Lösung. Nifedipin kann also solubilisiert werden. Bei Zugabe der im jeweiligen System erforderlichen Menge Wasser lässt sich auch ein entsprechendes Gel erhalten. In der folgenden Tabelle 3 wird $w_o$(gel) als dasjenige definiert, welches notwendig ist, um ein entsprechendes Gel zu erhalten.

Tabelle 3

| Fettsäureester | Nifedipin [mg/3ml] | $w_o$(gel) |
|---|---|---|
| Myristinsäureethylester | 32.1 | 5 |
| Myristinsäureisopropylester | 32.9 | 6 |
| Palmitinsäureisopropylester | 31.3 | 4 |
| Stearinsäurebutylester | 32.1 | 4 |

## Patentansprüche

1. Verfahren zur Herstellung von Gelen, auch hochviskose Massen genannt, dadurch gekennzeichnet, dass man durch Säulenchromatographie oder entsprechendes Verfahren gereinigtes Lecithin mit wenigstens einem organischen Lösungsmittel mischt, diese Suspension auf eine Temperatur bis 50 °C unter Rühren so lange erwärmt, bis sich wenigstens 50 Gew.-% des Lecithins gelöst haben, dann dem Gemisch Wasser in kleinen Mengen zugibt, bis die Lösung bei einer Kritischen Menge Wasser spontan erstarrt, und

   dass das Lecithin in einer Menge von 0,1 bis 30 Gew.-%, vorzugsweise 0,5 bis 15 Gew.-%, bezüglich dem organischen Lösungsmittel, zugegeben wird, und

   dass Wasser in einer Menge von 0,001 bis 30 Vol.-%, vorzugsweise von 0,001 bis 10 Vol.-%, bezüglich dem organischen Lösungsmittel, zugegeben wird.

2. Verfahren nach Anspruch 1, dadurch gekennzeichnet, dass das organische Lösungsmittel ausgewählt ist aus Alkanen, Estern, Aminen, einschliesslich dem halogenierten Homologen dieser Verbindungen, inklusive Perfluorverbindungen, Terpenen und pflanzlichen Oelen.

3. Verfahren nach einem der Ansprüche 1 bis 2, dadurch gekennzeichnet, dass das organische Lösungsmittel aus folgender Liste ausgewählt ist: Laurinsäureethylester, Laurinsäurebutylester, Myristinsäureethylester, Myristinsäureisopropylester, Palmitinsäureisopropylester, Stearinsäurebutylester, Isooktan, Cyclopentan, Cyclohexan, Cycloheptan, Cyclooktan, Cyclodekan, Methylcyclohexan, tert.-Butylcyclohexan, Bicyclohexyl, Phenylcyclohexan, 1,3,5-Triisopropylbenzol, Octylbenzol, trans-Dekalin, $CH_3(CH_2)_nCH_3$, wobei n eine ganze Zahl von 2 bis 15 bedeutet, 2,3-Dimethylbutan, 1-Hexen, 1-Okten, 1,7-Oktadien, (1R)-(+)-trans-Pinan, (1R)-(+)-cis-Pinan, Tripropylamin, Tributylamin, Triisobutylamin, Trioktylamin, N,N-Dioktylamin, Dibutylether , 2-Decenylbernsteinsäureanhydrid, Sonnenblumenöl und Olivenöl.

4. Verfahren nach einem der Ansprüche 1 bis 3, dadurch gekennzeichnet, dass man dem Gemisch vor der Erstarrung mindestens einen Wirkstoff zugibt, vorzugsweise ein Arzneimittel mit bewiesener oder vermuteter therapeutischer Wirkung; ein Vitamin oder ein Hormon; eine oder mehrere Aminosäuren; Coenzyme und phosphathaltige Biomoleküle wie Adenosintriphosphat, abgekürzt mit ATP, Adenosindiphosphat, abgekürzt mit ADP, oder Makromoleküle wie Proteine, darunter Enzyme; und Nukleinsäuren oder Polysaccharide, darunter Heparin, Stärke, Amylose; oder Bakterien, ganze Zellen oder Teile davon, vorzugsweise Mitochondrien; wie auch Farbstoffe, Salze, Metallionen; und auch organische Stoffe wie Glyzerin, Ethylenglykole, Zucker verschiedener Arten.

5. Verfahren nach Anspruch 4, dadurch gekennzeichnet, dass der Wirkstoff entweder in der wässrigen Phase, oder in der organischen Phase, oder in den Lecithinaggregaten lokalisiert ist, und insbesondere in einer Menge von 0,0001 bis 90 Gew.-%, bezogen auf Lecithin, vorhanden ist, vorzugsweise in einer Menge zwischen 0,0001 bis 30 Gew-%.

6. Verfahren nach einem der Ansprüche 1 bis 5, dadurch gekennzeichnet, dass Lecithin zum Teil durch andere Lipide, insbesondere Phosphatide, ersetzt wird, vorzugsweise durch Phosphatidsäure, Kephaline, Inositphosphatide, Plasmalogene, Sphingomyeline, Cerebroside, Sulfatide, Ganglioside.

7. Verfahren nach Anspruch 6, dadurch gekennzeichnet, dass der Phosphor enthaltende Stoff das genannte Lecithin in einer Menge von 0,1 bis 100 Gew.-%, insbesonders im Bereich von 10 bis 100 Gew.-%, ersetzt.

8. Verfahren nach einem der Ansprüche 1 bis 7, dadurch gekennzeichnet, dass Wasser durch andere Wasserstoffbrücken bildende Stoffe ersetzt wird, nämlich durch $D_2O$, Glyzerin, Ethylenglycol und dessen Polymere.

9. Verwendung der gemäss dem Verfahren nach einem der Ansprüche 1 bis 8 hergestellten Gele zur Herstellung von Formkörpern.

10. Verwendung nach Anspruch 9, dadurch gekennzeichnet, dass der Formkörper ein Gebrauchsgegenstand, vorzugsweise ein biokompatibler Behälter, eine Kapsel, eine Folie oder ein Film, ist.

**11.** Verwendung nach einem der Ansprüche 9 bis 10, dadurch gekennzeichnet, dass die Herstellung von Formkörpern mittels Verarbeitungsmethoden erfolgt, die man bei der Verarbeitung von Polymerschmelzen anwendet, insbesondere Formpressen und Filmgiessen.

**12.** Verwendung der gemäss dem Verfahren nach einem der Ansprüche 1 bis 8 hergestellten Gele in der Medizin oder Pharmakologie, insbesondere als pharmazeutische Präparate, oder in der Biotechnologie, vorzugsweise als Enzym- oder Zellenträger.

**13.** Gele, hergestellt gemäss dem Verfahren nach einem der Ansprüche 1 bis 8.

## Claims

**1.** A method for the preparation of gels, also named highly viscous masses, characterized by mixing lecithin, purified by column chromatography or by a corresponding process, with at least one organic solvent, heating this suspension to a temperature up to 50 °C under stirring during such a long time until at least 50 % by weight of the lecithin have dissolved, then adding water to the mixture in small portions until the solution solidifies spontaneously at a critical amount of water, and

that the lecithin is added in an amount from 0,1 to 30 % by weight, preferably from 0,5 to 15 % by weight, referred to the organic solvent, and

that the water is added in an amount from 0,001 to 30 volume -%, preferably from 0.001 to 10 volume -%, referred to the organic solvent.

**2.** The process according to claim 1, characterized in that the organic solvent is selected from alkanes, esters, amines, included the halogenated homologues of these compounds, included perfluoro compounds, terpenes and vegetable oils.

**3.** The process according to one of claims 1 to 2, characterized in that the organic solvent is selected from the following list: lauric acid ethyl ester, lauric acid butyl ester, myristic acid ethyl ester, myristic acid isopropyl ester, palmitic acid isopropyl ester, stearic acid butyl ester, isooctane, cyclopentane, cyclohexane, cycloheptane, cyclooctane, cyclodecane, methylcyclohexane, tert.-butylcyclohexane, bicyclohexyl, phenylcyclohexane, 1,3,5-triisopropylbenzene, octylbenzene, trans-decalin, $CH_3$ $(CH_2)_n$ $CH_3$ wherein n is an integr from 2 to 15, 2,3-dimethylbutane, 1-hexene, 1-octene, 1,7-octadiene, (1R)-(+)-trans-pinane, (1R)-(+)-cis-pinane, tripropylamine, tributylamine, triisobutylamine, trioctylamine, N,N-dioctylamine, dibutylether, 2-decenylsuccinic anhydride, sunflower oil and olive oil.

**4.** The process according to one of claims 1 to 3, characterized by adding to the mixture prior to the solidification at least one active component, preferably a medicament having a proven or presumed therapeutic activity; a vitamin or a hormone; one or more amino acid(s); coenzymes and phosphate containing biomolecules, such as adenosintriphosphate, abbreviated with ATP, adenosindiphosphate, abbreviated with ADP, or macromolecules, such as proteins, including enzymes; and nucleic acids or polysaccharides, including heparin, starch, amylose; or bacteria, whole cells or parts thereof, preferably mitochondria; as well as dyestuffs, salts, metal ions; and also organic compounds such as glycerin, ethylene glycols, sugars of different kinds.

**5.** The process according to claim 4, characterized in that the active component is located either in the aqueous phase or in the organic phase or in the lecithin aggregates, and is present especially in an amount from 0,0001 to 90 % by weight, preferably in an amount between 0,0001 to 30 % by weight, referred to lecithin.

**6.** The process according to one of claims 1 to 5, characterized in that the lecithin is replaced partly by other lipids, especially phosphatides, preferably phosphatidic acid, cephalines, inositphosphatides, plasmogenes, sphingomyelines, cerebrosides, sulfatides, gangliosides.

**7.** The process according to claim 6, characterized in that the phosphorous-containing compound replaces said lecithin in an amount from 0,1 to 100 % by weight, especially in the range from 10 to 100 % by weight.

8. The process according to one of claims 1 to 7, characterized in that the water is replaced by other hydrogen bridges forming compounds, namely $D_2O$, glycerin, ethylene glycol and its polymers.

9. Use of the gels prepared according to the process according to one of claims 1 to 8 for the preparation of molded articles.

10. Use according to claim 9, characterized in that the molded article is a commodity, preferably a biocompatible container, a capsule, a foil or a film.

11. Use according to one of claims 9 to 10, characterized in that the preparation of molded articles is realized by means of processing methods, which are used in the processing of polymer melts, especially compression molding and film casting.

12. Use of the gels, prepared according to the process according to one of claims 1 to 8 in the medicine or in the pharmacology, especially as pharmaceutical preparations, or in the biotechnology, preferably as support for enzymes or cells.

13. Gels, prepared according to the process according to one of claims 1 to 8.

## Revendications

1. Procode de préparation de gels, également appelés masses très visqueuses, caractérisé en ce qu'on mélange de la lécithine, purifiée par chromatographie sur colonne ou par des procédés correspondants, avec au moins un solvant organique, on chauffe cette suspension sous agitation à une température de 50°C au maximum jusqu'à ce qu'au moins 50% en poids de la lécithine se soient dissous, puis on ajoute au mélange de l'eau en petites quantités jusqu'à ce que la solution se prenne en masse spontanément pour une quantité critique d'eau,
   en ce que la lécithine est ajoutée dans une proportion de 0,1 à 30% en poids, de préférence de 0,5 à 15% en poids, par rapport au solvant organique, et
   en ce que l'eau est ajoutée dans une proportion de 0,001 à 30% en volume, de préférence de 0,001 à 10% en volume, par rapport au solvant organique.

2. Procédé selon la revendication 1, caractérisé en ce que le solvant organique est choisi parmi les alcanes, les esters, les amines, y compris les homologues halogénés de ces composés, notamment les composés perfluorés, les terpènes et les huiles végétales.

3. Procédé selon la revendication 1 ou 2, caractérisé en ce que le solvant organique est choisi dans la liste suivante: laurate d'éthyle, laurate de butyle, myristate d'éthyle, myristate d'isopropyle, palmitate d'isopropyle, stéarate de butyle, isooctane, cyclopentane, cyclohexane, cycloheptane, cyclooctane, cyclodécane, methylcyclohexane, tert.-butylcyclohexane, bicyclohexyle, phénylcyclohexane, 1,3,5-triisopropylbenzène, octylbenzène, trans-décaline, $CH_3(CH_2)_nCH_3$, n représentant un nombre entier de 2 à 15, 2,3-diméthylbutane, 1-hexène, 1-octène, 1,7-octadiène, (1R)-(+)-trans-pinane, (1R)-(+)-cis-pinane, tripropylamine, tributylamine, triisobutylamine, trioctylamine, N,N-dioctylamine, dibutyléther, anhydride 2-décénylsuccinique, huile de tournesol et huile d'olive.

4. Procédé selon l'une quelconque des revendications 1 à 3, caractérisé en ce qu'on ajoute au mélange, avant sa prise en masse, au moins une substance active, de préférence un médicament à activité thérapeutique prouvée ou présumée; une vitamine ou une hormone; un ou plusieurs acides aminés; des co-enzymes et des biomolécules phosphatées, telles que le triphosphate d'adénosine désigné par l'abréviation ATP, le diphosphate d'adénosine désigne par l'abréviation ADP, ou des macromolécules telles que des protéines et notamment des enzymes; et des acides nucléiques ou des polysaccharides, notamment l'héparine, l'amidon, l'amylose; ou des bactéries, des cellules entières ou des parties de celles-ci, de préférence des mitochondries; ainsi que des colorants, des sels, des ions metalliques; et même des substances organiques telles que la glycérine, les éthylèneglycols, les sucres de différents types.

5. Procédé selon la revendication 4, caractérisé en ce que la substance active est localisée soit dans la phase aqueuse, soit dans la phase organique, soit dans les agrégats de lécithine, et est présente en

particulier dans une proportion de 0,0001 à 90% en poids par rapport à la lécithine, de préférence dans une proportion comprise entre 0,0001 et 30% en poids.

6. Procédé selon l'une quelconque des revendications 1 à 5, caractérisé en ce que la lécithine est remplacée en partie par d'autres lipides, en particulier des phosphatides, de préférence par l'acide phosphatidique, les céphalines, les inosite-phosphatides, les plasmalogènes, les sphingomyélines, les cérébrosides, les sulfatides, les gangliosides.

7. Procédé selon la revendication 6, caractérisé en ce que la substance contenant du phosphore remplace ladite lécithine a raison de 0,1 à 100% en poids, en particulier à raison de 10 à 100% en poids.

8. Procédé selon l'une quelconque des revendications 1 à 7, caractérisé en ce que l'eau est remplacée par d'autres substances formant des ponts hydrogène, à savoir par $D_2O$, la glycérine, l'éthylèneglycol et des polymères de celui-ci.

9. Utilisation des gels préparés par le procédé selon l'une quelconque des revendications 1 à 8 pour la fabrication de corps moulés.

10. Utilisation selon la revendication 9, caractérisée en ce que le corps moulé est un objet usuel, de préférence un récipient biocompatible, une capsule, une feuille ou un film.

11. Utilisation selon la rèvendication 9 ou 10, caractérisée en ce que la fabrication de corps moulés est effectuée par des procédés de transformation que l'on applique dans la transformation de masses fondues de polymères, en particulier le moulage par compression et la coulée des films.

12. Utilisation des gels préparés par le procédé selon l'une quelconque des revendications 1 à 8 en médecine ou en pharmacologie, en particulier comme préparations pharmaceutiques, ou dans la biotechnologie, de préférence comme support d'enzymes ou de cellules.

13. Gels préparés par le procédé selon l'une quelconque des revendications 1 à 8.

Fig. 1

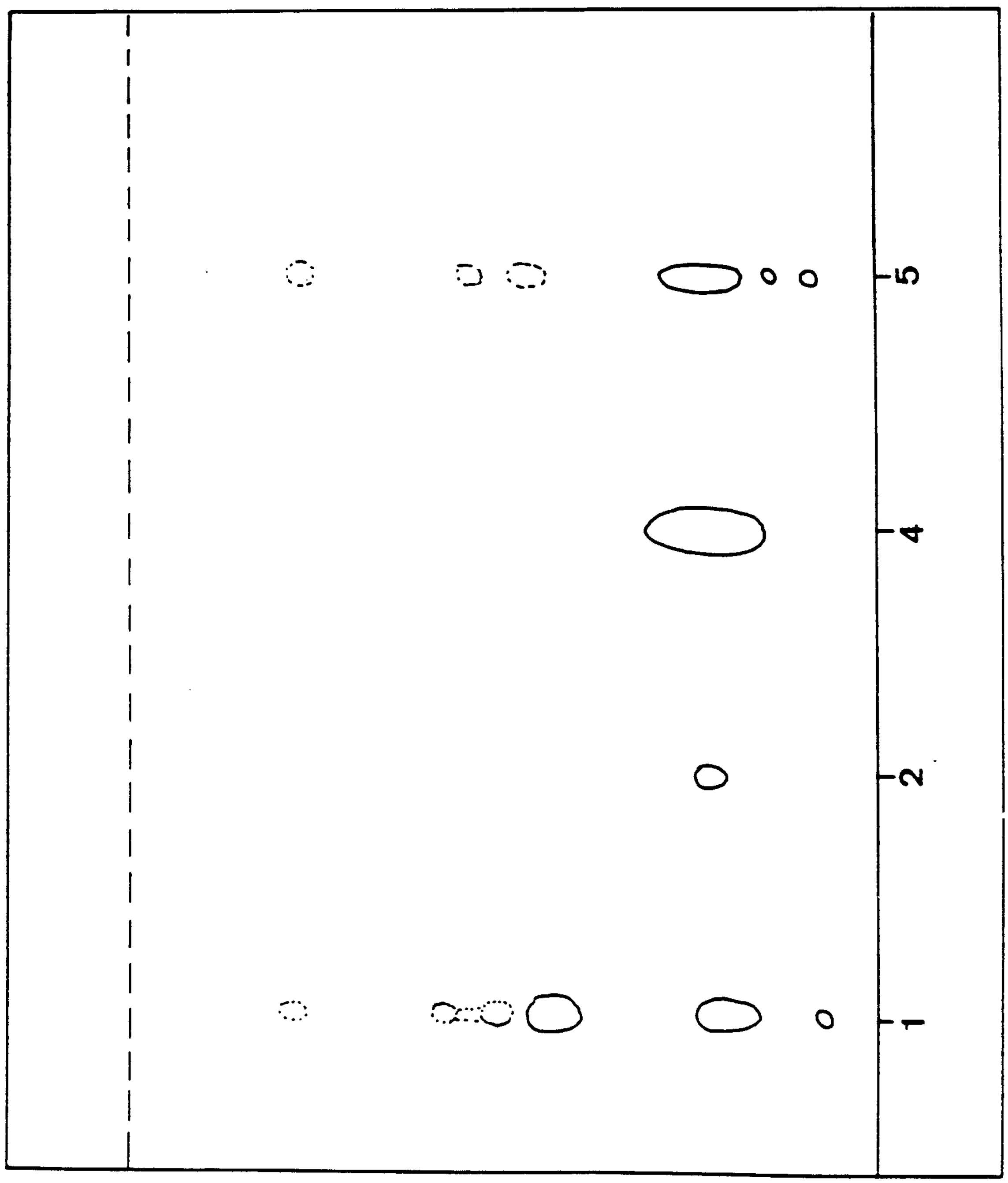

Fig. 2